# FASCICULE DE BREVET EUROPEEN

(11) **EP 2 984 990 B1**
(45) Date de publication et mention de la délivrance du brevet: **08.11.2017**
(21) Numéro de dépôt: 15175460.3
(22) Date de dépôt: 06.07.2015
(51) Int. Cl.: A61B 8/08, A61B 8/00

(54) **DISPOSITIF DE VISUALISATION DE L'ANGLE DE CLÔTURE MÉDIO URÉTRAL DE L'URÈTRE DE L'URÈTRE FÉMININ À L'AIDE DE IMAGERIE PAR RÉSONANCE MAGNÉTIQUE NUCLÉAIRE OU PAR ÉCHOGRAPHIE**
VORRICHTUNG ZUM ANZEIGEN DES MEDIO-URETHRALEN VERSCHLUSSWINKELS DER WEIBLICHEN HARNRÖHRE MITHILFE EINES BILDGEBENDEN VERFAHRENS DURCH KERNSPINRESONANZ ODER ULTRASCHALL
DEVICE FOR VIEWING THE MID-URETHRAL ANGLE OF CLOSURE OF THE FEMALE URETHRA USING NUCLEAR MAGNETIC RESONANCE IMAGING OR ULTRASOUND IMAGING

(30) Priorité: 11.08.2014 FR 1457739
(43) Date de publication de la demande: 17.02.2016
(73) Titulaire: Eurl Cornier, 75116 Paris (FR)
(72) Inventeur: CORNIER, Edgard, 75116 Paris (FR)
(74) Mandataire: Cabinet HERRBURGER

(56) Documents cités:
- WO-A1-96/02214
- US-A1- 2003 114 901
- US-A1- 2009 005 645

## Description

La présente invention a pour objet un dispositif de visualisation de l'angle de clôture médio urétral de l'urètre féminin à l'aide de l'imagerie par résonnance magnétique nucléaire ou par échographie pour permettre d'évaluer les risques de fuites urinaires.

Chez la femme, l'urètre, qui est le canal de sortie de la vessie présente la particularité d'être très court, environ 6 cm pour une largeur d'environ 3 à 4 mm.

Il existe très fréquemment des problèmes de fuites urinaires pouvant s'avérer très invalidantes auxquelles les spécialistes cherchent à remédier, en règle générale par des méthodes chirurgicales consistant en particulier à mettre en place des bandelettes sous-urétrales permettant de suspendre l'urètre à sa partie moyenne (techniques dites de Mouchel et TVT).

Les spécialistes ont pendant longtemps considéré que la continence urinaire chez la femme était contrôlée par le sphincter de la vessie qui est un muscle situé à la jonction de la vessie et de l'urètre et que des sollicitations trop importantes ou des défauts de ce sphincter étaient seuls à l'origine des flux urinaires.

Par suite, pour évaluer le risque de fuites urinaires et donc la nécessité d'un traitement, les spécialistes utilisent classiquement la cystomanométrie qui correspond à une mesure de la pression que ce sphincter exerce pour fermer l'urètre, ou pression de clôture ; une telle mesure est effectuée en passant une sonde de pression dans l'urètre de la patiente.

Tout se passe ainsi comme si le sphincter devait être positionné dans une enceinte de pression équilibrée assurant la continence urinaire du fait de la seule fermeture ou ouverture du fragile sphincter de la vessie.

On a toutefois du établir que 21 % des femmes continentes le sont avec un sphincter vésical ouvert (réf. : Chapple et Coll - 1989 ; in : René RIOU - CHU Henri Mondor ; anatomie et physiologie de la continence Urinaire - APHP- Google) de sorte que la théorie selon laquelle l'enceinte de pression manométrique du col de la vessie assurerait la continence est peu probable.

La plicature du fond de la vessie sur l'urètre, à hauteur de la jonction avec le col vésical a été supposé aider à la fermeture de l'urètre. L'étude de l'angle entre la paroi postérieure de la vessie et l'urètre, appelé aussi angle sphinctérien, ou aussi angle urétral, et les dispositifs décrits pour mesurer cet angle entre l'urètre et le fond de la vessie dans la zone du sphincter, n'ont pas trouvé d'intérêt diagnostique et n'ont pas prospéré à l'usage (systèmes de chainettes placées dans la vessie et l'urètre).

Prenant acte du succès thérapeutique de la mise en place des bandelettes sous urétrales, à la partie moyenne ou antérieure de l'urètre, donc a distance du sphincter, pour guérir les incontinences urinaires d'effort, et la comparant au geste du jardinier qui plie son tuyau pour arrêter le jet, sans fermeture du robinet-sphincter, on a été amené conformément à l'invention à supposer qu'une théorie dynamique, en rapport avec l'anatomie de l'urètre féminin peut permettre de concilier la mobilité physiologique de l'utérus, du vagin avec la continence urinaire (réf. : The Uterus Swing : Coelioscopic Correction of Pelvic Prolapse ; article proposé et refusé - No. EJORB-12-9117, 2013).

L'anatomie décrit classiquement l'urètre féminin comme un organe creux, aplati et à cavité virtuelle, dont le trajet anatomique derrière le pubis est concave vers l'avant du haut vers le bas.

Des études anatomiques plus précises compliquent la description classique trop simple et montrent un rétrécissement de l'organe au niveau de la zone du tiers moyen de l'urètre, et du sphincter strié urétral. De Lancey (1986), Umek et Coll (2003) ont pu décrire cinq segments fonctionnels et de trajet anatomiquement différents de l'urètre féminin.

L'IRM 3D Cube sans préparation peut préciser les plans successifs exacts du trajet de l'urètre féminin. Elle permet de voir que, chez la femme jeune, l'urètre au repos n'est pas rectiligne ni concave mais est orienté en zigzag.

L'urètre se dirige tout d'abord en bas vers la face supérieure du vagin depuis le col de la vessie (urètre vaginal) avant de se diriger en bas et en avant, vers le pubis (urètre rétro pubien). A ce niveau se situent les ligaments pubo-urétraux moyens provoquent le rétrécissement médian de la configuration en forme de diabolo de l'urètre récemment décrite.

L'urètre se dirige ensuite vers le bas mais un peu plus vers le vagin en arrière, puis en s'enroulant sous le bord inférieur du pubis en direction du méat urétral (urètre proximal).

Cette anatomie a conduit à définir l'existence d'un trajet que l'invention nomme le zigzag urétral, et l'invention nomme un angle de clôture urétral, c'est-à-dire l'angle que dessinent les deux premiers segments de l'urètre, de la vessie vers l'arrière du pubis, puis de l'arrière du pubis vers le vagin sous pubien. Ces définitions (zigzag urétral et angle de clôture medio urétral) sont des définitions nouvelles et innovantes. Elles ne sont pas décrites dans les traités d'anatomie.

On a été amené à supposer que l'anatomie en zigzag de l'urètre chez la jeune femme est fragile, qu'il peut évoluer avec l'âge, la survenue de grossesse ou des traitements médicamenteux tels que la corticothérapie.

Toutefois, l'exploration de cet organe creux est très mal aisée. Retrouver la forme en zigzag, ou non de l'urètre nécessite un matériel coûteux (L 'IRM 3D Cube), une recherche longue et délicate, du fait de la multiplication des coupes à un niveau de définition très élevé. Les variations anatomiques sont nombreuses. Tout ceci rend la seule exploration difficile par la seule imagerie, sans avoir recours à un moyen simple de repérage de l'urètre à examiner.

L'urétrographie per mictionnelle est difficile à réaliser et interpréter dans la mesure où les incidences de profil strict sont peu aisées en condition de miction sous IRM ou Echographie.

De plus, la mise en place de chainettes métalliques telle qu'elle a déjà été proposée a travers des tubes rigides ou non n'a pas donné les résultats escomptés et ne peut pas être utile en IRM, et la mise en place d'une sonde arrondie relativement rigide modifie l'anatomie du fait de la grande élasticité des ligaments retro pubiens.

La présente invention a pour objet de proposer un dispositif de visualisation de l'angle de clôture medio urétral de l'urètre féminin à l'aide de l'imagerie par résonnance magnétique nucléaire ou par échographie, tel que défini par la revendication 1, pour permettre d'évaluer les risques de fuites urinaires.

Selon l'invention, un tel dispositif est constitué par un élément de visualisation destiné à être introduit dans l'urètre et dans la vessie, puis à y être positionné, et à y être maintenu avant un examen d'imagerie médicale.

Selon l'invention, cet élément de visualisation se prolonge à son extrémité distale par un élément de maintien dans la vessie.

Selon une caractéristique de l'invention, le dispositif de visualisation comprend également un élément de positionnement destiné à être introduit dans l'urètre et dans la vessie et ayant pour fonction de permettre l'introduction et le positionnement de l'élément de visualisation dans l'urètre et dans la vessie avant un examen d'imagerie médicale.

Selon l'invention, l'élément de visualisation est réalisé en un matériau souple stérilisable et biocompatible sans mémoire de forme et repérable par résonance magnétique nucléaire ou par échographie.

L'élément de positionnement est quant à lui réalisé en un matériau rigide stérilisable et biocompatible.

L'élément de maintien est soit un ballonnet, soit une forme enroulée.

L'élément de visualisation et/ou l'élément de positionnement correspondent avantageusement à un élément à usage unique particulièrement peu onéreux.

Selon l'invention, l'élément de visualisation est avantageusement marqué par des striations repérables par résonance magnétique nucléaire ou par échographie, en particulier susceptible de pouvoir être distingué par l'imagerie IRM 3D Cube.

Plus précisément et selon une première variante de l'invention, l'élément de visualisation est constitué par un cathéter comportant une partie proximale sans mémoire de forme destinée à être positionnée dans l'urètre qui se prolonge à son extrémité distale par une partie de maintien à mémoire de forme enroulée destinée à être positionnée dans la vessie.

Selon cette première variante de l'invention, l'élément de positionnement est quant à lui constitué par une sonde creuse comportant une extrémité distale à bout mousse ou olivaire ouverte. Cette sonde creuse constitue un canal de guidage longitudinal ouvert à ses deux extrémités et destiné à recevoir le cathéter pour permettre sa mise en place dans l'urètre et dans la vessie.

La sonde de positionnement a en particulier une longueur de 10 à 20 cm tandis que le canal de guidage de celle-ci a un diamètre de 8 à 10 charrières ou 2 à 4 mm.

Le cathéter de visualisation sans mémoire de forme a avantageusement une longueur essentiellement égale au double de la longueur de la sonde.

La partie de maintien distale à mémoire de forme de ce cathéter a, quant à elle, une longueur de l'ordre de 2 cm à l'état déroulé. Cette partie de maintien est solidaire de la partie « sans mémoire de forme » du cathéter de visualisation. Elle est préparée introduite déroulée - rectiligne dans la sonde de positionnement, préalablement à l'examen de la patiente. Il suffit qu'elle soit poussée hors de la sonde d'introduction pour qu'elle passe de la forme rectiligne à la forme enroulée du fait de la particularité du matériau à mémoire de forme.

Selon cette première variante de l'invention, le cathéter est préalablement introduit dans le canal de guidage de la sonde, de sorte que la partie de maintien distale à mémoire de forme de celui-ci ne dépasse pas l'extrémité distale de cette sonde et que sa partie de visualisation proximale sans mémoire de forme soit libre dans la sonde, et l'ensemble ainsi constitué peut avantageusement être stérilisé dans un sachet fermé.

Conformément à cette première variante de l'invention, avant l'examen d'imagerie, le praticien place la sonde de positionnement dans l'urètre de la patiente après nettoyage antiseptique du méat (sondage urinaire classique), puis la pousse jusque dans la vessie. Il exerce alors une traction sur l'extrémité proximale du cathéter de positionnement et en même temps avec l'autre main repousse le cathéter de visualisation, vers l'intérieur de la vessie de sorte que la partie distale à mémoire de forme de celui-ci soit repoussée à l'intérieur de la sonde, et dans la vessie. Progressivement la sonde de positionnement est retirée cependant que le cathéter de visualisation est repoussé. Sa partie distale à mémoire de forme se place dans la vessie ; la partie proximale sans mémoire de forme épouse le trajet de l'urètre. Ce trajet est ainsi repérable pour les examens d'imagerie.

Lorsque la sonde de positionnement a été totalement retirée, seule reste dans la vessie la partie enroulée à mémoire de forme du cathéter de visualisation qui indique la zone vésicale, tandis que la partie proximale souple sans mémoire de forme de ce cathéter de visualisation est positionnée dans l'urètre de la patiente où elle peut se déformer de façon à prendre automatiquement sa forme en zigzag pour permettre l'examen par résonance magnétique nucléaire ou par échographie et donc la mesure de l'angle de clôture de l'urètre proximal de la patiente.

Après cet examen, ce cathéter peut facilement être retiré de l'urètre de la patiente par le praticien.

Selon une seconde variante de l'invention, l'élément de visualisation est constitué par une gaine tubulaire fermée à son extrémité distale. Elle est destinée à être positionnée dans l'urètre avec à son extrémité distale un ballonnet de maintien destiné à être positionné dans la vessie et susceptible d'être rempli par du sérum physiologique pour être gonflé et maintenu dans la vessie.

Ce ballonnet de maintien peut avantageusement avoir une contenance de 3 à 10 ml. Un fin tuyau de remplissage du ballonnet est notamment solidaire de l'élément de visualisation. Il est constitué lui aussi d'un matériel sans mémoire de forme. Son extrémité porte un embout Luer qui permet de raccorder une seringue.

L'élément de positionnement est quant à lui constitué par un guide rigide pouvant être en métal ou en matière plastique destiné à être introduit à la partie interne de la gaine tubulaire pour permettre sa mise en place dans l'urètre et dans la vessie.

Selon cette seconde variante de l'invention, le guide rigide est préalablement introduit dans la gaine tubulaire, et l'ensemble ainsi constitué peut avantageusement être stérilisé dans un sachet fermé.

Pour permettre de réaliser l'examen radiologique, la gaine tubulaire ainsi équipée du guide rigide est introduite par le praticien dans l'urètre et la vessie de la patiente par son extrémité distale, après désinfection du méat urétral. Le praticien remplit ensuite le ballonnet par du sérum physiologique de façon à permettre de gonfler ce ballonnet pour qu'il reste en place dans la vessie.

Après cette mise en place, le praticien retire le guide rigide de l'urètre de la patiente.

La gaine tubulaire est ainsi, à la fois positionnée et maintenue dans l'urètre de la patiente où elle peut se déformer de façon à prendre automatiquement sa forme en zigzag pour permettre l'examen par résonance magnétique nucléaire ou par échographie et donc la mesure de l'angle de clôture médio urétral de l'urètre proximale de la patiente.

Après cet examen, le ballonnet peut facilement être dégonflé et la gaine tubulaire retirée de l'urètre de la patiente par le praticien.

Selon la troisième variante de l'invention, le ballonnet de maintien n'est pas collé sur la périphérie externe de la gaine tubulaire mais fait partie intégrante de cette gaine qu'il prolonge à son extrémité distale. La partie distale de la gaine est constituée par un matériau plus fin, ou plus élastique, sans mémoire de forme que le matériau constituant le reste de la sonde de visualisation. La cathéter de placement rigide destiné à être temporairement introduit dans la sonde pour la guider vers l'intérieur de la vessie est le même que pour les versions précédentes.

Selon cette variante, le ballonnet de maintien 11 est gonflé par le canal intérieur de la gaine tubulaire 10 qui peut être fermé par un bouchon non représenté.

Les caractéristiques du dispositif qui fait l'objet de l'invention seront décrites plus en détail en se référant aux dessins non limitatifs annexés dans lesquels :
- la figure 1a est un schéma représentant la sonde de positionnement du dispositif conforme à la première variante de l'invention,
- la figure 1b est un schéma représentant le cathéter de visualisation de ce dispositif,
- les figures 2a et 2b représentent le cathéter de visualisation mis en place dans la sonde dans deux positions différentes,
- la figure 3 est un schéma représentant le dispositif conforme à la première variante de l'invention mis en place dans l'urètre et dans la vessie d'une patiente,
- la figure 4 est un schéma représentant ce même dispositif après ablation de la sonde de positionnement,
- les figures 5a, 5b et 5c sont des schémas représentant respectivement les éléments d'un dispositif conforme à la seconde variante de l'invention,
- les figures 6a et 6b sont des schémas représentant ce dispositif dans deux positions différentes,
- la figure 7 est un schéma représentant le dispositif conforme à la seconde variante de l'invention mis en place dans l'urètre et dans la vessie d'une patiente,
- la figure 8 est un schéma analogue à la figure 7 mais représentant le dispositif après extraction du guide rigide et gonflement du ballonnet de maintien, et
- les figures 9a et 9b sont des schémas correspondant aux figures 6a et 6b mais représentant une troisième variante de l'invention.

Il est à noter que sur les figures les éléments du dispositif sont représentés à une échelle fortement agrandie.

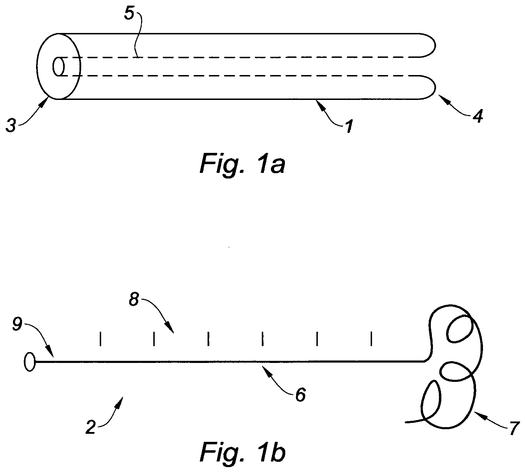

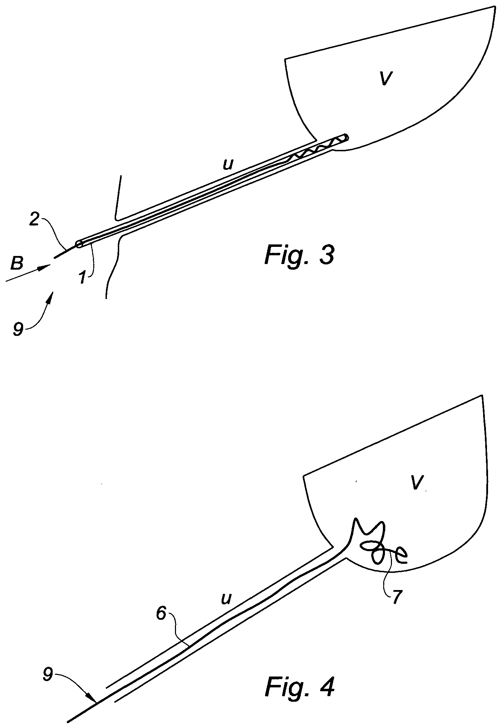

Selon les figures 1 à 4, le dispositif conforme à la première variante de l'invention est constitué par une sonde de positionnement 1 représentée sur la figure 1a qui est destinée à être introduite dans l'urètre U et dans la vessie V d'une patiente comme schématisé sur la figure 3 ainsi que par un cathéter de visualisation 2 représenté sur la figure 1b qui est lui aussi destiné à être introduit dans l'urètre U et dans la vessie V d'une patiente comme schématisé sur les figures 3 et 4 avant un examen radiologique pour permettre d'effectuer cet examen.

Selon la figure 1a la sonde 1 est constituée par un élément ayant une longueur de 10 à 20 centimètres qui est réalisé en un matériau stérilisable et biocompatible et comporte une extrémité proximale 3 et une extrémité distale 4 à bout mousse ou olivaire.

La sonde de positionnement 1 est percée d'un canal de guidage longitudinal 5 ouvert à ses deux extrémités et ayant un diamètre de 8 à 10 charrières ou 2 à 4 millimètres.

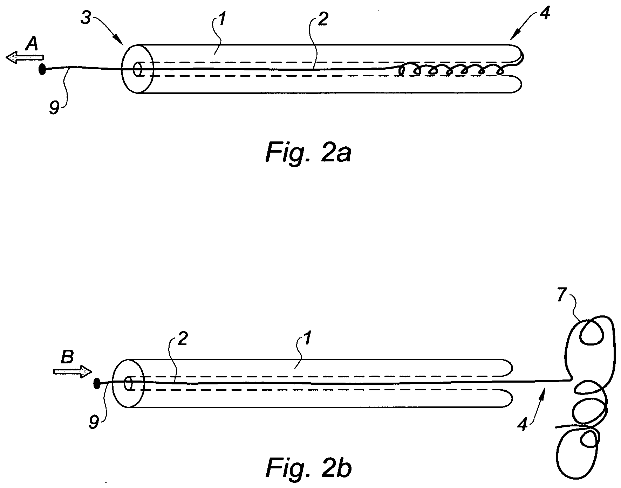

Ce canal de guidage 5 est destiné à recevoir le cathéter de visualisation 2 comme représenté sur les figures 2a et 2b pour permettre sa mise en place dans l'urètre U et dans la vessie V d'une patiente, comme représenté sur les figures 3 et 4.

Selon la figure 1b, le cathéter 2 est constitué par un élément non radio-opaque souple ayant une longueur approximativement égale au double de la longueur de la sonde 1 qui comporte une partie proximale 6 sans mémoire de forme destinée à être positionnée dans l'urètre U d'une patiente et une partie distale 7 à mémoire de forme, de forme enroulée destiné à être positionnée dans la vessie V d'une patiente, comme schématisé sur la figure 4.

La partie proximale 6 du cathéter 2 est marquée par des striations repérables par résonance magnétique nucléaire 8 qui sont schématisées sur la figure 1b.

Il est à noter que la partie distale 7 du cathéter 2 a une longueur de l'ordre de 2 centimètres à l'état déroulé.

Le cathéter 2 est préalablement introduit dans la sonde 1 de sorte que sa partie distale 7 de forme arrondie dépasse de l'extrémité distale 4 de celle-ci et l'ensemble ainsi constitué est stérilisé dans un sachet fermé non représenté sur les figures.

Selon les figures 2a et 2b, avant la mise en place de l'ensemble constitué par la sonde 1 et le cathéter de visualisation 2, le praticien exerce une traction schématisée par la flèche A (figure 2a) au niveau de l'extrémité proximale 9 du cathéter 2 qui dépasse au niveau de l'extrémité proximale 3 de la sonde 1 de sorte que la partie distale souple de forme enroulée 7 de ce cathéter 2 pénètre à la partie interne du canal de guidage 5 de la sonde 1 au niveau de l'extrémité distale 4 de cette sonde de visualisation en devenant temporairement essentiellement rectiligne comme représenté sur la figure 2a.

Selon la figure 3, pour permettre de réaliser l'examen radiologique, l'ensemble constitué par la sonde 1 et le cathéter 2 peut être introduit dans cet état par le praticien dans l'urètre U et la vessie V de la patiente.

Après cette mise en place, le praticien exerce une poussée schématisée par la flèche B au niveau de l'extrémité proximale 9 du cathéter 2 de façon à repousser la partie distale 7 de ce cathéter 2 dans la vessie V de la patiente de sorte qu'elle reprenne sa forme enroulée initiale comme représenté sur les figures 2b et 4.

Avant de pratiquer l'examen d'imagerie médicale, le praticien exerce une traction sur la sonde de positionnement pour la retirer, en même temps qu'une contre-poussée sur le cathéter de visualisation afin qu'il reste positionné durant le retrait de la sonde.

Il retire ensuite la sonde 1 de sorte que seul le cathéter 2 reste dans l'urètre U et dans la vessie V de la patiente comme représenté sur la figure 4.

Il est à noter qu'après ablation de la sonde 1, la partie proximale 6 du cathéter 2 n'est pas rectiligne mais prend automatiquement la forme en zigzag de l'urètre U qui n'a pas été représentée sur la figure 4 dans un but de clarté.

Grâce à la présence des striations 8 repérables par résonance magnétique nucléaire, ce cathéter 2 permet de décrire, après repérages de ces striations les rapports anatomiques de l'urètre U de la patiente et le respect ou non de son angle de clôture médio urétral derrière l'os pubien.

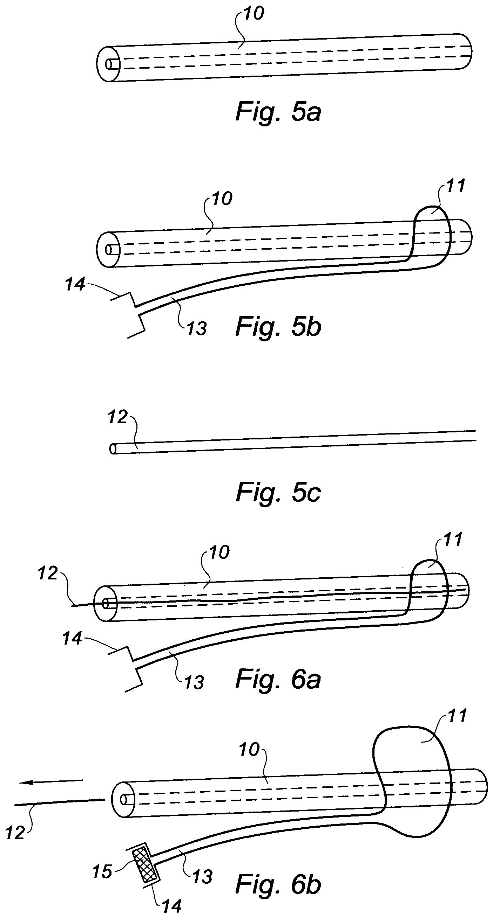

Selon les figures 5a, 5b, 5c, 6a, 6b, 7 et 8, le dispositif conforme à la seconde variante de l'invention est constitué par une gaine tubulaire souple 10 représentée sur la figure 5a marquée ou non marquée par des striations repérables par résonance magnétique nucléaire qui ne sont pas représentées, et à l'extrémité distale de laquelle est collé un ballonnet de maintien 11 relié à un tuyau de remplissage 13 muni d'un embout luer pour seringue 14 à son extrémité libre comme représenté sur la figure 5b.

Cette gaine tubulaire 10 est rigidifiée par un guide rigide 12 introduit à travers sa partie proximale comme représenté sur les figures 5c et 6a.

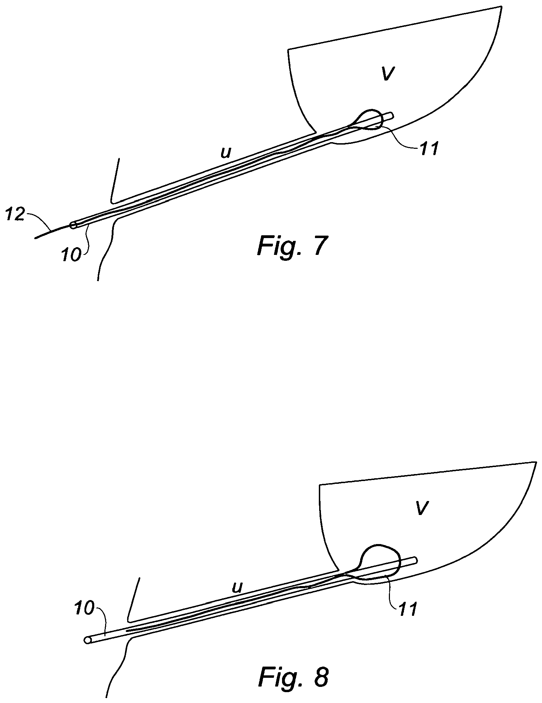

L'ensemble ainsi constitué est introduit dans l'urètre U et dans la vessie d'une patiente avant un examen d'imagerie médicale pour permettre d'effectuer cet examen comme représenté sur les figures 6a et 7.

Le ballonnet de maintien est gonflé avec 3 à 5 cc de sérum physiologique par l'intermédiaire du tuyau de remplissage 13 et- de l'embout luer 14 comme représenté sur les figures 6b et 8 puis l'embout luer est fermé par un bouchon 15 représenté sur la figure 6b.

Après cette introduction, la gaine rigide d'introduction 12 est extraite de la gaine tubulaire de visualisation 10 avec les mêmes manoeuvres médicales de poussée et de contre-poussée que précédemment décrites de sorte que seule reste dans l'urètre et dans la vessie de la patiente cette gaine tubulaire de visualisation 10 qui est positionnée dans l'urètre ainsi que le ballonnet 11 collé à l'extrémité distale de celle-ci qui est positionné dans la vessie V.

Pour permettre le maintien de cet ensemble 10, 11 ainsi positionné, du sérum physiologique est introduit dans le tuyau de remplissage 13 par l'embout luer 14 de façon à gonfler le ballonnet 11.

Il est à noter qu'après extraction du guide rigide 12, la gaine tubulaire 10 n'est pas rectiligne mais prend automatiquement la forme en zigzag de l'urètre U qui n'a pas été représentée sur la figure 7 dans un but de clarté.

On mesure ainsi l'angle de clôture médio urétral.

Grâce à la présence des striations repérables par résonance magnétique nucléaire qui ne sont pas représentées, cette gaine tubulaire 10 permet ainsi sur des images d'IRM 3D Cube de décrire après repérage de ces striations les rapports anatomiques de l'urètre U de la patiente et le respect ou non de son angle de clôture médio urétral derrière l'os pubien.

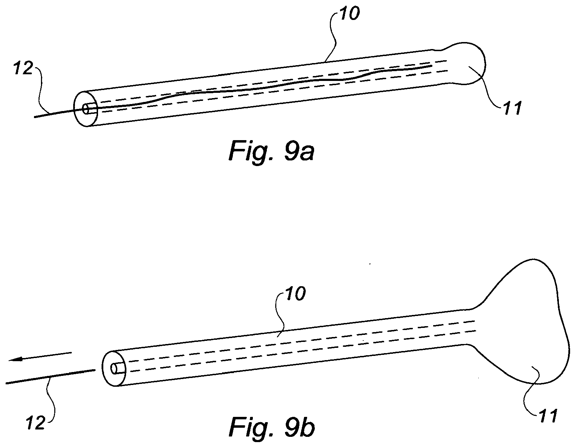

Selon la troisième variante de l'invention représentée sur les figures 9a et 9b, le ballonnet de maintien 11 n'est pas collé sur la périphérie externe de la gaine tubulaire 10 mais fait partie intégrante de cette gaine qu'il prolonge à son extrémité distale.

Selon cette variante, le ballonnet de maintien 11 est gonflé par le canal intérieur de la gaine tubulaire 10 qui peut être fermé par un bouchon non représenté.

### NOMENCLATURE

- 1.: Sonde
- 2.: Cathéter
- 3.: Extrémité proximale de la sonde
- 4.: Extrémité distale de la sonde
- 5.: Canal de guidage
- 6.: Partie proximale du cathéter
- 7.: Partie distale du cathéter
- 8.: Striations
- 9.: Extrémité proximale du cathéter
- 10.: Gaine tubulaire
- 11.: Ballonnet
- 12.: Guide rigide
- 13.: Tuyau de remplissage
- 14.: Embout luer
- 15.: Bouchon

- U: Urètre
- V: Vessie

## Revendications

1. Dispositif de visualisation de l'angle de clôture médio urétral de l'urètre féminin à l'aide de l'imagerie par résonance magnétique nucléaire ou par échographie pour permettre d'évaluer les risques de fuites urinaires,
ce dispositif est constitué par :
- un élément de visualisation (2, 10) réalisé en un matériau souple stérilisable et biocompatible sans mémoire de forme et repérable par résonnance magnétique nucléaire ou par échographie sur toute sa longueur destiné à être introduit dans l'urètre (U) et dans la vessie (V), à y être positionné et y être maintenu avant un examen d'imagerie médicale, et à se déformer de façon à prendre automatiquement la forme en zigzag de l'urètre au repos pour permettre la mesure de l'angle de clôture médio urétral,
- un élément de maintien dans la vessie (7, 11), prolongeant l'élément de visualisation (2, 10) à son extrémité distale, et
- un élément de positionnement (1, 12) réalisé en un matériau rigide stérilisable et biocompabible destiné à être introduit dans l'urètre (U) et dans la vessie (V) pour permettre l'introduction et le positionnement de l'élément de visualisation (2, 10) et de l'élément de maintien (7, 11) avant un examen d'imagerie médicale, et à être retiré avant cet examen.

2. Dispositif selon la revendication 1,
**caractérisé en ce que**
l'élément de visualisation (2, 10) et/ou l'élément de positionnement (1, 12) est (sont) à usage unique.

3. Dispositif selon l'une quelconque des revendications 1 et 2, **caractérisé en ce que**
l'élément de visualisation (2, 10) est marqué par des striations (8) repérables par résonance magnétique nucléaire ou par échographie.

4. Dispositif selon l'une quelconque des revendications 1 à 3, **caractérisé en ce que**
l'élément de visualisation est constitué par un cathéter (2) comportant une partie proximale sans mémoire de forme (6) destinée à être positionnée dans l'urètre (U) se prolongeant à son extrémité distale par un élément de maintien à mémoire de forme (7), de forme enroulée, destiné à être positionné dans la vessie (V) et
l'élément de positionnement est constitué par une sonde creuse (1) comportant une extrémité distale (4) à bout mousse ou olivaire ouverte à ses deux extrémités constituant un canal de guidage longitudinal (5) destiné à recevoir le cathéter (2) pour permettre sa mise en place dans l'urètre (U) et dans la vessie (V).

5. Dispositif selon la revendication 4,
**caractérisé en ce que**
la sonde de positionnement (1) a une longueur de 10 à 20 cm et le canal de guidage (5) de celle-ci a un diamètre de 8 à 10 charrières ou 2 à 4 mm.

6. Dispositif selon l'une quelconque des revendications 4 et 5, **caractérisé en ce que**
le cathéter de visualisation (2) a une longueur essentiellement égale au double de la longueur de la sonde (1) et l'élément de maintien distal (7) a une longueur de l'ordre de 2 cm à l'état déroulé.

7. Dispositif selon l'une quelconque des revendications 1 à 3, **caractérisé en ce que**
l'élément de visualisation est constitué par une gaine tubulaire (10) destinée à être positionnée dans l'urètre (U) et portant à sont extrémité distale un ballonnet de maintien (11) constituant l'élément de maintien destiné à être positionné dans la vessie (V) et susceptible d'être rempli par du sérum physiologique pour être gonflé et maintenu dans la vessie (V), et
l'élément de positionnement est constitué par un guide rigide (12) destiné à être introduit à la partie interne de la gaine tubulaire (10) pour permettre sa mise en place dans l'urètre (U) et dans la vessie (V).

8. Dispositif selon la revendication 7,
**caractérisé en ce que**
le ballonnet de maintien (11) a une contenance de 3 à 10 ml.

## Patentansprüche

1. Vorrichtung zur Visualisierung des medio-urethralen Verschlusswinkels der weiblichen Harnröhre mittels Bildgebung durch magnetische Kernresonanz oder durch Ultraschall, um eine Beurteilung des Risikos eines Harnverlustes zu ermöglichen,
wobei diese Vorrichtung aus Folgendem besteht:
- einem Visualisierungselement (2, 10) aus einem flexiblen sterilisierbaren und biokompatiblen Material ohne Formgedächtnis, das durch magnetische Kernresonanz oder Ultraschall über seine gesamte Länge lokalisierbar ist und dazu bestimmt ist, vor einer bildgebenden medizinischen Untersuchung in die Harnröhre (U) und in die Harnblase (V) eingeführt zu werden, darin positioniert zu werden und darin gehalten zu werden und sich so zu verformen, dass es automatisch die Zickzackform der Harnröhre im Ruhezustand annimmt, um die Messung des medio-urethralen Verschlusswinkels zu ermöglichen,
- einem Element zum Halten in der Blase (7, 11), welches das Visualisierungselement (2, 10) an seinem distalen Ende verlängert, und
- einem Positionierungselement (1, 12), das aus einem starren sterilisierbaren und biokompatiblen Material hergestellt ist und dazu bestimmt ist, in die Harnröhre (U) und in die Harnblase (V) eingeführt zu werden, um die Einführung und Positionierung des Visualisierungselements (2, 10) und des Halteelements (7, 11) vor einer bildgebenden medizinischen Untersuchung zu ermöglichen, und vor dieser Untersuchung herausgezogen zu werden.

2. Vorrichtung nach Anspruch 1,
**dadurch gekennzeichnet, dass**
das Visualisierungselement (2, 10) und/oder das Positionierungselement (1, 12) zum Einmalgebrauch bestimmt ist (sind).

3. Vorrichtung nach irgendeinem der Ansprüche 1 und 2,
**dadurch gekennzeichnet, dass**
das Visualisierungselement (2, 10) mit Streifen (8) markiert ist, die durch magnetische Kernresonanz oder Ultraschall lokalisierbar sind.

4. Vorrichtung nach irgendeinem der Ansprüche 1 bis 3,
**dadurch gekennzeichnet, dass**
das Visualisierungselement aus einem Katheter (2) besteht, der einen proximalen Teil ohne Formgedächtnis (6) aufweist, welcher dazu bestimmt ist, in der Harnröhre (U) positioniert zu werden, und der sich an seinem distalen Ende in einem Halteelement mit Formgedächtnis (7) von gewundener Form fortsetzt, das dazu bestimmt ist, in der Harnblase (V) positioniert zu werden, und
das Positionierungselement aus einer Hohlsonde (1) besteht, die ein distales Ende (4) mit Schaumstoffspitze oder olivenförmiger Spitze aufweist, an ihren beiden Enden offen ist und einen längslaufenden Führungskanal (5) bildet, der dazu bestimmt ist, den Katheter (2) aufzunehmen, um dessen Platzierung in der Harnröhre (U) und in der Harnblase (V) zu ermöglichen.

5. Vorrichtung nach Anspruch 4,
**dadurch gekennzeichnet, dass**
die Positionierungssonde (1) eine Länge von 10 bis 20 cm aufweist und der Führungskanal (5) von dieser einen Durchmesser von 8 bis 10 Charriere oder 2 bis 4 mm aufweist.

6. Vorrichtung nach irgendeinem der Ansprüche 4 und 5,
**dadurch gekennzeichnet, dass**
der Visualisierungskatheter (2) eine Länge aufweist, die im Wesentlichen der doppelten Länge der Sonde (1) entspricht, und das distale Halteelement (7) im entrollten Zustand eine Länge in der Größenordnung von 2 cm aufweist.

7. Vorrichtung nach irgendeinem der Ansprüche 1 bis 3,
**dadurch gekennzeichnet, dass**
das Visualisierungselement aus einer röhrenförmigen Hülse (10) besteht, die dazu bestimmt ist, in der Harnröhre (U) positioniert zu werden, und an ihrem distalen Ende einen Halteballon (11) trägt, der das Halteelement bildet, das dazu bestimmt ist, in der Harnblase (V) positioniert zu werden, und mit isotonischer Kochsalzlösung gefüllt werden kann, um aufgepumpt und in der Harnblase (V) gehalten zu werden, und
das Positionierungselement aus einer starren Führung (12) besteht, die dazu bestimmt ist, in den inneren Teil der röhrenförmigen Hülse (10) eingeführt zu werden, um deren Platzierung in der Harnröhre (U) und in der Harnblase (V) zu ermöglichen.

8. Vorrichtung nach Anspruch 7,
**dadurch gekennzeichnet, dass**
der Halteballon (11) ein Fassungsvermögen von 3 bis 10 ml aufweist.

## Claims

1. Device for displaying the mid-urethral angle of closure of the female urethra using nuclear magnetic resonance imaging or by means of echography in order to allow an evaluation of the risks of urine leakages,
this device is constituted by:
- a display element (2, 10) which is produced from a flexible material which can be sterilised and which is biocompatible without shape memory and which can be identified by means of nuclear magnetic resonance or by means of echography over the entire length thereof and which is intended to be introduced into the urethra (U) and into the bladder (V), to be positioned at that location and to be retained at that location before a medical imaging examination, and to be deformed in order to automatically assume the zigzag shape of the urethra in the rest state in order to enable the measurement of the mid-urethral angle of closure,
- a retention element (7, 11) in the bladder, extending the display element (2, 10) at the distal end thereof, and
- a positioning element (1, 12) which is produced from a rigid sterilisable and biocompatible material which is intended to be introduced into the urethra (U) and into the bladder (V) in order to enable the introduction and the positioning of the display element (2, 10) and the retention element (7, 11) before a medical imaging examination and to be removed before this examination.

2. Device according to claim 1,
**characterised in that**
the display element (2, 10) and/or the positioning element (1, 12) is/are for a single use.

3. Device according to either claim 1 or claim 2, **characterised in that**
the display element (2, 10) is marked by striations (8) which can be identified by nuclear magnetic resonance or by means of echography.

4. Device according to any one of claims 1 to 3, **characterised in that**
the display element is constituted by a catheter (2) which comprises a proximal portion without shape memory (6) which is intended to be positioned in the urethra (U) and which is extended at the distal end thereof by a shape-memory retention element (7) which is of rolled up form and which is intended to be positioned in the bladder (V), and
the positioning element is constituted by a hollow probe (1) which comprises a distal end (4) with a blunt or open olive-like end at the two ends thereof and which constitutes a longitudinal guiding channel (5) which is intended to receive the catheter (2) in order to enable it to be positioned in the urethra (U) and in the bladder (V).

5. Device according to claim 4,
**characterised in that**
the positioning probe (1) has a length of from 10 to 20 cm and the guiding channel (5) thereof has a diameter of from 8 to 10 charriers or 2 to 4 mm.

6. Device according to either claim 4 or 5,
**characterised in that**
the display catheter (2) has a length which is substantially equal to double the length of the probe (1) and the distal retention element (7) has a length in the order of 2 cm in the unrolled state.

7. Device according to any one of claims 1 to 3, **characterised in that**
the display element is constituted by a tubular sheath (10) which is intended to be positioned in the urethra (U) and which carries at the distal end thereof a retention balloon (11) which constitutes the retention element which is intended to be positioned in the bladder (V) and which is capable of being filled by physiological serum in order to be inflated and retained in the bladder (V), and
the positioning element is constituted by a rigid guide (12) which is intended to be introduced at the inner portion of the tubular sheath (10) in order to enable it to be positioned in the urethra (U) and in the bladder (V).

8. Device according to claim 7
**characterised in that**
the retention balloon (11) has a capacity of from 3 to 10 ml.
